# EUROPEAN PATENT APPLICATION

(11) **EP 3 202 947 A1**
(43) Date of publication of application: **09.08.2017**
(21) Application number: 17153087.6
(22) Date of filing: 25.01.2017
(51) Int. Cl.: C23C 14/02, A61L 2/238, C23C 14/16, C23C 14/34, C23C 28/02

(54) **METHOD FOR DEPOSITING PVD GERM REPELLENT FILM**

(30) Priority: 04.02.2016 CN 201610078676
(71) Applicant: Dongguan Shatou Asahi Metal Electronic Parts Co., Ltd., Dongguan City, Guangdong 518132 (CN)
(72) Inventor: LAI, Kin Man, Dongguan City, Guangdong 518132 (CN)
(74) Representative: ZHAOffice SPRL

(57) **Abstract**

Disclosed is method for plating a PVD (physical vapor deposition) germ killing film, employing a vacuum magnetron sputtering technology and using a nano-silver-containing target for uniformly distributing nano-silver to form a germ killing film. The method can achieve the aim of full germ killing. Besides, nano-silver is enveloped in the sputtering target to form the film, so the target material target can play the role of protecting the nano-silver. Target material can be Al, Cr, stainless steel and Cu. Therefore, the nano-silver is protected; wear resistance of the germ killing film is increased; and the germ killing film can continuously kills germs for a long time.

## Description

### BACKGROUND OF THE INVENTION

### Technical Field

The present invention relates to a method for plating a PVD film, in particular to a method for plating a PVD
germ killing film.

### 2. Description of Related Art

PVD (Physical Vapor Deposition) refers to a process for physical mass transfer by which atoms or molecules are transferred from a source to the surface of a base material. During PVD operation, different metal vapors can be employed. The metal vapors are ionized to be in an electron state. Ions are led to workpieces by a bias voltage to deposit and form a film. Before depositing on the workpieces, the ions may react and combine with other ions to generate composite films which are different in hardness, brightness, friction coefficient, color, etc. to meet functional or appearance requirements.

Affected by factors such as environment pollution, many articles that people touch in daily life usually carry a huge amount of germs, becoming germ pollution sources and disease spreading sources. Therefore, development of plated products with
germ killing performance has a very important significance for the improvement of the living environment and protection of the health of people. Nano-silver has been verified to have strong restraining and disinfection roles on dozens of common pathogenic bacteria in life, and not generate drug resistance, so nano-silver is now being widely used in daily life.

In the prior art, when nano-silver is used to kill germs, nano-silver is usually mixed with other materials to protect the surfaces of workpieces as a coating, or the surfaces of the workpieces are plated with films containing nano-silver. The methods all have defects such as non-uniform nano-silver distribution, insufficient wear resistance of the coating, and failure to continuously killing germs for a long time.

### BRIEF SUMMARY OF THE INVENTION

The objective of the present invention is to provide a method for plating a PVD germ killing film to solve the problems of non-uniform nano-silver distribution and failure to continuously kill germs for a long time of the existing germ killing film.

A method for plating a PVD germ killing film includes the following steps:
1) pre treatment: washing the surface of a workpiece clean, removing an oxide layer, placing the workpiece in a vacuum chamber, vacuuming the chamber, and raising the temperature;
2) generation of a base layer: starting a Ti arc target, pumping Ar, loading a bias voltage, and depositing a Ti base film on the surface of the workpiece;
3) generation of a germ killing film: turn off the Ti arc target, starting an Al sputtering target containing not greater than 10% nano-silver, pumping Ar, loading the bias voltage, and depositing the germ killing film on the surface of the workpiece.

In one of the embodiments, the method also comprises the steps of, after the depositing of the germ killing film, turn off the Al-Ag sputtering targets, turning off power, gradually pressurizing the vacuum chamber, reducing the temperature to be not greater than 70°C, and then taking out the workpiece.

In one of the embodiments, in step 1) the pretreatment also comprises pumping Ar to perform ion cleaning on the workpiece for 20min after vacuuming.

In one of the embodiments, the film plating process employed in both step 2 and step 3) is a vacuum magnetron sputtering plating process.

In one of the embodiments, in step 1) the vacuuming proceeds until the intensity of the pressure is in the range of 4.0×10⁻³∼6.0×10⁻³Pa.

In one of the embodiments, in step 1) the temperature is raised to be 100∼150°C.

In one of the embodiments, in step 2) Ar is pumped so that the intensity of the pressure in the chamber is in the range of 2∼5 Pa; and in step 3) Ar is pumped so that the intensity of the pressure in the chamber is maintained in the range of 0.2∼0.5 Pa.

In one of the embodiments, in step 2) the loaded bias voltage is -400V - -600V; and in step 3) the loaded bias voltage is -50V--100V.

In one of the embodiments, in step 2) the depositing time is 8∼15 min; and in step 3) the depositing time is 30∼50 min.

A method for plating a PVD (physical vapor deposition) germ killing film employs a vacuum magnetron sputtering PVD process to ensure uniform distribution of the nano-silver. The method can achieve the aim of full germ killing. Besides, nano-silver is enveloped in the targets to be plated on a film, so the Al material of the Al-Ag target can play the role of protecting the nano-silver. Therefore, the nano-silver is protected; wear resistance of the germ killing film is increased; and the germ repellent film can repel germs for a long time.

### DETAILED DESCRIPTION OF THE INVENTION

To make the objectives, technical scheme and advantages of the present invention more clear, the present invention is described in further detail in conjunction with the following embodiments. It should be understood that the embodiments depicted here are only used for better explaining the technical scheme of the present invention, not for limiting the present invention.

A method for plating a PVD germ killing film includes the following steps:
1) pretreatment: washing the surface of a workpiece clean, removing an oxide layer, placing the work piece in a vacuum chamber, vacuuming the chamber, and raising temperature;
2) generation of a base layer: starting a Ti arc target, pumping Ar, loading a bias voltage, and depositing a Ti base film on the surface of the workpiece;
3) generation of a germ killing film: turn off the Ti arc target, starting an Al sputtering target containing not greater than 10% nano-silver, pumping Ar, loading the bias voltage, and depositing the germ killing film on the surface of the workpiece.

The layer of Ti film deposited can activate the surface of the workpiece, improve the surface energy, and increase the adhesion between the film and the surface of the workpiece. Here, the Al sputtering target can be used to plate an Al film.

In the germ killing film, the nano-silver plays the germ killing role. The germ killing film is plated with the Al sputtering target containing the nano-silver, so on the one hand, Al can protect the nano-silver to avoid the nano-silver from directly contacting the air and being easily oxidized by 02 in the air to be inactive, and on the other hand, Al can increase the wear resistance of the germ killing film and can achieve the aim of germ killing for a long time. If the content of the nano-silver in the target exceeds 10%, on the one hand, the cost of the germ killing film increases because of the price of the nano-silver, and on the other hand, due to the high nano-silver content, many nano-silver particles are directly coated on the surface of the germ killing film, affecting the wear resistance of the germ killing film, so the content of the nano-silver in the target does not exceed 10%. Further preferably, the content of the nano-silver in the target is 3%-6%. Besides, copper with the same germ killing effect can be used to replace the nano-silver.

In one of the embodiments, the method also comprises the steps of, after the depositing of the film, turning off the sputtering targets, turning off the power, gradually pressurizing the vacuum chamber, reducing the temperature to be not greater than 70°C, and then taking out the workpiece. If the temperature is too high, after the film is taken out of the vacuum chamber, substances in the film are easily oxidized by the oxygen in the air, affecting the quality of the PVD film.

In one of the embodiments, in step 1) the pretreatment also comprises pumping Ar to perform ion cleaning on the workpiece for 20min after vacuuming. On the one hand, ion cleaning ensures high cleanliness. On the other hand, ion cleaning motivates the activation energy of the surface of the workpiece and can increase of the adhesion force of the plated film.

In one of the embodiments, the film plating process employed in both step 2 and step 3) is a vacuum magnetron sputtering plating process. Magnetron sputtering can be used for preparing various materials such as metals, semiconductors and insulators, and has the advantages of simple equipment, a large plating area and a strong adhesion.

In one of the embodiments, in step 1) the vacuuming proceeds until the intensity of the pressure is in the range of 4.0×10⁻³∼ 6.0×10⁻³Pa.

In one of the embodiments, in step 1) the temperature is raised to be 100∼150°C.

In one of the embodiments, in step 2) Ar is pumped so that the intensity of the pressure in the chamber is in the range of 2∼5 Pa; and in step 3) Ar is pumped so that the intensity of the pressure in the furnace is in the range of 0.2∼0.5 Pa. When Ar is pumped, Ar ions can bombard the target surface such that the target sputters ions; during particle sputtering, neutral target atoms or molecules deposit on the workpiece to form a film.

In one of the embodiments, in step 2) the loaded bias voltage is -400V - -600V; and in step 3) the loaded bias voltage is -50V- -100V.

In one of the embodiments, in step 2) the depositing time is 8∼15 min; and in step 3) the depositing time is 30∼50 min.

The method of the present invention can be used for plating the PVD germ killing film on various materials such as copper alloy, zinc alloy and stainless steel.

### Embodiment 1

1) The surface of a workpiece is washed clean to remove the oxide layer and then placed in a vacuum chamber; the chamber is vacuumed until the intensity of pressure reaches 4.0×10⁻³Pa; and Ar is pumped into the furnace to perform ion cleaning for 20 min.
2) The temperature in the chamber is raised to 100°C.
3) A Ti arc target is started; Ar is pumped such that the intensity of the pressure in the furnace reaches 2Pa; a bias voltage of -400V is loaded; and then a Ti base layer is deposited on the surface of the workpiece, wherein the depositing time is 8 min.
4) The Ti arc target is turned off; an Al sputtering target containing 10% nano-silver is started; Ar is pumped such that the intensity of the pressure in the chamber reaches 0.2Pa; a bias voltage of -50V is loaded; and then a germ killing film is deposited on the surface of the workpiece, wherein the depositing time is 50 min. 5) The sputtering target is turned off, and all other power are also turned off; the vacuum chamber is gradually pressured while the temperature in the furnace reduces to 65°C, and then the workpiece is taken out.

### Embodiment 2

1) The surface of a workpiece is washed clean to remove the oxide layer and then placed in a vacuum chamber; the chamber is vacuumed until the intensity of pressure reaches 6.0×10⁻³Pa; and Ar is pumped into the chamber to perform ion cleaning for 20 min.
2) The temperature in the furnace is raised to 150°C.
3) A Ti arc target is started; Ar is pumped such that the intensity of the pressure in the furnace reaches 5Pa; a bias voltage of -600V is loaded; and then a Ti base layer is deposited on the surface of the workpiece, wherein the depositing time is 15 min.
4) The Ti arc target is closed; an Al sputtering target containing 6% of nano-silver is started; Ar is pumped such that the intensity of the pressure in the furnace reaches 0.5Pa; a bias voltage of -100V is loaded; and then a germ killing film is deposited on the surface of the workpiece, wherein the depositing time is 30 min.
5) The sputtering target is turned off, and all power is also turned off; the vacuum furnace is gradually pressured while the temperature in the furnace reduces to 65°C, and then the workpiece is taken out.

### Embodiment 3

1) The surface of a workpiece is washed clean to remove the oxide layer and then placed in a vacuum chamber; the chamber is vacuumed until the intensity of the pressure reaches 5.0×10⁻³Pa; and Ar is pumped into the chamber to perform ion cleaning for 20 min.
2) The temperature in the chamber is raised to 110°C.
3) A Ti arc target is started; Ar is pumped such that the intensity of the pressure in the furnace reaches 3Pa; a bias voltage of -400V is loaded; and then a Ti base layer is deposited on the surface of the workpiece, wherein the depositing time is 10 min.
4) The Ti arc target is turned off; an Al sputtering target containing 3% of nano-silver is started; Ar is pumped such that the intensity of the pressure in the chamber reaches 0.4Pa; a bias voltage of -80V is loaded; and then a germ killing film is deposited on the surface of the workpiece, wherein the depositing time is 35 min.
5) The sputtering target is turned off, and all other power also turned off; the vacuum chamber is gradually pressured while the temperature in the chamber reduces to 60°C, and then the workpiece is taken out.

### Embodiment 4

1) The surface of a workpiece is washed clean to remove the oxide layer and then placed in a vacuum chamber; the chamber is vacuumed until the intensity of pressure reaches 4.0×10⁻³Pa; and Ar is pumped into the chamber to perform ion cleaning for 20min.
2) The temperature in the chamber is raised to 120°C.
3) A Ti arc target is started; Ar is pumped such that the intensity of the pressure in the furnace reaches 3Pa; a bias voltage of -450V is loaded; and then a Ti base layer is deposited on the surface of the workpiece, wherein the depositing time is 12 min.
4) The Ti arc target is closed; an Al sputtering target containing 5% of nano-silver is started; Ar is pumped such that the intensity of the pressure in the furnace reaches 0.3Pa; a bias voltage of -60V is loaded; and then a germ killing film is deposited on the surface of the workpiece, wherein the depositing time is 40 min.
5) The sputtering target is closed, and all other power turned off; the vacuum chamber is gradually pressured while the temperature in the chamber reduces to 65°C, and then the workpiece is taken out.

### Embodiment 5

1) The surface of a workpiece is washed clean to remove the oxide layer and then placed in a vacuum chamber; the chamber is vacuumed until the intensity of the pressure reaches 5.0×10⁻³Pa; and Ar is pumped into the chamber to perform ion cleaning for 20min.
2) The temperature in the chamber is raised to 130°C.
3) A Ti arc target is started; Ar is pumped such that the intensity of the pressure in the chamber reaches 5Pa; a bias voltage of -550V is loaded; and then a Ti base layer is deposited on the surface of the workpiece, wherein the depositing time is 13 min.
4) The Ti arc target is turned off; an Al sputtering target containing 2% of nano-silver is started; Ar is pumped such that the intensity of the pressure in the furnace reaches 0.5Pa; a bias voltage of -50V is loaded; and then a germ killing film is deposited on the surface of the workpiece, wherein the depositing time is 45 min.
5) The sputtering target is turned off, and all other power turned off; the vacuum chamber is gradually pressured while the temperature in the chamber reduces to 65°C, and then the workpiece is taken out.

### Embodiment 6

1) The surface of a workpiece is washed clean to remove the oxide layer and then placed in a vacuum chamber; the chamber is vacuumed until the intensity of the pressure reaches 6.0×10⁻³Pa; and Ar is pumped into the chamber to perform ion cleaning for 20 min.
2) The temperature in the chamber is raised to 150°C.
3) A Ti arc target is started; Ar is pumped such that the intensity of the pressure in the chamber reaches 3Pa; a bias voltage of -470V is loaded; and then a Ti base layer is deposited on the surface of the workpiece, wherein the depositing time is 8 min.
4) The Ti arc target is turned off; an Al sputtering target containing 10% nano-silver is started; Ar is pumped such that the intensity of the pressure in the chamber reaches 0.3Pa; a bias voltage of -90V is loaded; and then a germ killing film is deposited on the surface of the workpiece, wherein the depositing time is 30 min.
5) The sputtering target is turned off; and all power other turned off; the vacuum chamber is gradually pressured while the temperature in the chamber reduces to 70°C, and then the workpiece is taken out.

### Embodiment 7

1) The surface of a workpiece is washed clean to remove the oxide layer and then placed in a vacuum chamber; the chamber is vacuumed until the intensity of the pressure reaches 4.0×10⁻³Pa; and Ar is pumped into the chamber to perform ion cleaning for 20 min.
2) The temperature in the chamber is raised to 110°C.
3) A Ti arc target is started; Ar is pumped such that the intensity of the pressure in the chamber reaches 2Pa; a bias voltage of -400V is loaded; and then a Ti base layer is deposited on the surface of the workpiece, wherein the depositing time is 15min.
4) The Ti arc target is turned off; an Al sputtering target containing 1% of nano-silver is started; Ar is pumped such that the intensity of the pressure in the chamber reaches 0.2Pa; a bias voltage of -100V is loaded; and then a germ killing film is deposited on the surface of the workpiece, wherein the depositing time is 30 min.
5) The sputtering target is turned off and all other power turned off; the vacuum chamber is gradually pressured while the temperature in the chamber reduces to 65°C, and then the workpiece is taken out.

### Embodiment 8

1) The surface of a workpiece is washed clean to remove the oxide layer and then placed in a vacuum chamber; the chamber is vacuumed until the intensity of the pressure reaches 4.0×10⁻³Pa; and Ar is pumped into the chamber to perform ion cleaning for 20 min.
2) The temperature in the chamber is raised to 100°C.
3) A Ti arc target is started; Ar is pumped such that the intensity of the pressure in the chamber reaches 5Pa; a bias voltage of -600V is loaded; and then a Ti base layer is deposited on the surface of the workpiece, wherein the depositing time is 11 min.
4) The Ti arc target is turned off; an Al sputtering target containing 0.5% of nano-silver is started; Ar is pumped such that the intensity of the pressure in the furnace reaches 0.2Pa; a bias voltage of -80V is loaded; and then a germ killing film is deposited on the surface of the workpiece, wherein the depositing time is 40 min.
5) The sputtering target is turned off, and all other power turned off; the vacuum chamber is gradually pressured while the temperature in the chamber reduces to 65°C, and then the workpiece is taken out.

### Embodiment 9

1) The surface of a workpiece is washed clean to remove the oxide layer and then placed in a vacuum chamber; the chamber is vacuumed until the intensity of the pressure reaches 5.0×10⁻³Pa; and Ar is pumped into the furnace to perform ion cleaning for 20min.
2) The temperature in the chamber is raised to 130°C.
3) A Ti arc target is started; Ar is pumped such that the intensity of the pressure in the chamber reaches 3Pa; a bias voltage of -500V is loaded; and then a Ti base layer is deposited on the surface of the workpiece, wherein the depositing time is 8 min.
4) The Ti arc target is turned off; an Al sputtering target containing 0.2% of nano-silver is started; Ar is pumped such that the intensity of the pressure in the chamber reaches 0.5Pa; a bias voltage of -50V is loaded; and then a germ killing film is deposited on the surface of the workpiece, wherein the depositing time is 50min.
5) The sputtering target is closed, and all other power turned off; the vacuum chamber is gradually pressured while the temperature in the chamber reduces to 70°C, and then the workpiece is taken out.

The above embodiments are just preferable embodiments of the present invention. It should be noted that, for those ordinarily skilled in this field, improvements and changes can be made on the basis of the principle of the present invention. The improvements and changes shall also fall within the protective scope of the present invention.

## Claims

1. A method for plating a PVD germ killing film, **characterized by** comprising the following steps:
1) pretreatment: washing the surface of a workpiece clean, removing an oxide layer, placing the work piece in a vacuum chamber, vacuuming the chamber, and raising the temperature;
2) generation of a base layer: starting a Ti arc target, pumping Ar, loading a bias voltage, and depositing a Ti base film on the surface of the workpiece;
3) generation of a germ killing film: turn off the Ti arc target, starting an Al sputtering target containing not greater than 10% nano-silver, pumping Ar, loading the bias voltage, and depositing the germ killing film on the surface of the workpiece.

2. The method for plating a PVD germ killing film according to claim 1, **characterized in that**, the method also comprises the steps of, after the depositing of the germ killing film, turn off the sputtering targets, turning off all power, gradually pressurizing the vacuum chamber, reducing the temperature to be not greater than 70°C, and then taking out the workpiece.

3. The method for plating a PVD germ killing film according to claim 1, **characterized in that**, in step 1) the pretreatment also comprises pumping Ar to perform ion cleaning on the workpiece for 20 min after vacuuming.

4. The method for plating a PVD germ killing film according to claim 1, **characterized in that**, the film plating process employed in both step 2) and step 3) is a vacuum magnetron sputtering plating process.

5. The method for plating a PVD germ killing film according to claim 1, **characterized in that**, in step 1) the vacuuming proceeds until the intensity of the pressure is in the range of 4.0x10⁻³∼6.0×10⁻³Pa.

6. The method for plating a PVD germ killing film according to claim 1, **characterized in that**, in step 1) the temperature is raised to be 100∼150°C.

7. The method for plating a PVD germ killing film according to claim 1, **characterized in that**, in step 2) Ar is pumped so that the intensity of the pressure in the chamber is in the range of 2∼5 Pa; and in step 3) Ar is pumped so that the intensity of the pressure in the chamber is in the range of 0.2∼0.5 Pa.

8. The method for plating a PVD germ killing film according to claim 1, **characterized in that**, in step 2) the loaded bias voltage is -400V ∼ -600V; and in step 3) the loaded bias voltage is -50V∼ -100V.

9. The method for plating a PVD germ killing film according to claim 1, **characterized in that**, in step 2) the depositing time is 8∼15 min; and in step 3) the depositing time is 30∼50min.
